# FASCICULE DE BREVET EUROPEEN

(11) **EP 1 274 389 B1**
(45) Date de publication et mention de la délivrance du brevet: **05.07.2006**
(21) Numéro de dépôt: 01917202.2
(22) Date de dépôt: 21.03.2001
(51) Int. Cl.: A61Q 5/10, A61K 8/41, A61K 8/49, A61K 8/73, A61K 8/81

(54) **COMPOSITION POUR LA TEINTURE D'OXYDATION DES FIBRES KERATINIQUES COMPRENANT UN DERIVE DE LA 3,5-DIAMINO-PYRIDINE ET UN POLYMERE EPAISSISSANT PARTICULIER**
ZUSAMMENSETZUNG FÜR DIE OXIDATIVE FÄRBUNG VON KERATINISCHEN FASERN, DIE EIN 3,5-DIAMINPYRIDINDERIVAT UND EIN BESONDERES VERDICKUNGSPOLYMER ENTHÄLT
OXIDATION DYEING COMPOSITION FOR KERATINOUS FIBRES COMPRISING A 3,5-DIAMINO-PYRIDINE DERIVATIVE AND A PARTICULAR THICKENING POLYMER

(30) Priorité: 12.04.2000 FR 0004721
(43) Date de publication de la demande: 15.01.2003
(73) Titulaire: L'ORÉAL, 75008 Paris (FR)
(72) Inventeur: AUDOUSSET, Marie-Pascale, F-92600 Asnières (FR)
(74) Mandataire: Wattremez, Catherine
(86) Numéro de dépôt international: PCT/FR2001/000846
(87) Numéro de publication internationale: WO 2001/078668

(56) Documents cités:
- EP-A- 1 138 318
- DE-A- 4 018 335
- US-A- 4 698 065
- US-A- 4 923 977
- US-A- 5 279 616
- US-A- 5 743 919
- US-B- 4 473 375

## Description

La présente invention concerne une composition pour la teinture d'oxydation des fibres kératiniques, en particulier des fibres kératiniques humaines telles que les cheveux, comprenant, dans un milieu approprié pour la teinture, au moins un coupleur choisi parmi les dérivés de la 3,5-diamino-pyridine et leurs sels d'addition avec un acide, au moins un précurseur de colorant d'oxydation, et au moins un polymère épaississant particulier défini ci-après.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abimée) entre sa pointe et sa racine.

Ainsi, il a déjà été proposé, notamment dans las brevets US-4473375 ou DE-3132885 des compositions de teinture d'oxydation contenant certains dérivés de la 3,5-diamino-pyridine à titre de coupleur, en association avec des bases d'oxydation classiquement utilisées en teinture d'oxydation.
Cependant, les colorations obtenues en mettant en oeuvre ces compositions ne sont pas toujours assez puissantes, chromatiques, ou résistantes aux différentes agressions que peuvent subir les cheveux.

Il est de même connu dans la demande de brevet EP 1138318, de mettre en oeuvre une composition prête à l'emploi pour la coloration de fibres kératiniques, comprenant dans un milieu approprié pour la teinture, au moins un colorant d'oxydation choisi parmi des dérivés particuliers d'amino-alcoxy pyridines, et au moins un agent oxydant de nature enzymatique.

Or, la Demanderesse vient maintenant de découvrir qu'il est possible d'obtenir de façon totalement inattendue et surprenante, de nouvelles teintures d'oxydation, capables de conduire à des colorations aux nuances variées, chromatiques, puissantes, esthétiques, peu sélectives, et résistant bien aux diverses agressions que peuvent subir les fibres, en associant au moins un coupleur choisi parmi les 3,5-diamino-pyridirles de formule (I) définie ci-après et leurs sels d'addition avec un acide, au moins un précurseur de colorant d'oxydation, et au moins un polymère épaississant particulier défini ci-après.

Cette découverte est à la base de la présente invention.

L'invention a donc pour premier objet une composition pour la teinture d'oxydation des fibres kératiniques et en particulier des fibres kératiniques humaines telles que les cheveux, comprenant dans un milieu approprié pour la teinture,
(a) **à titre de coupleur**, au moins un dérivé de 3,5-diamino-pyridine de formule (I) suivante :
   <a> 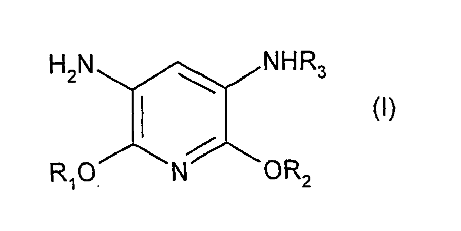 dans laquelle :
   - R₁ et R₂, identiques ou différents, représentent un radical alkyle en C₁-C₄, monohydroxyalkyle en C₁-C₄, ou polyhydroxyalkyle en C₂-C₄,
   - R₃ représente un atome d'hydrogène, un radical alkyle en C₁-C₄, monohydroxyalkyle en C₁-C₄ ou polyhydroxyalkyle en C₂-C₄
   ou l'un de ses sels d'addition avec un acide ;
   et,
(b) au moins un précurseur de colorant d'oxydation,
   caractérisée par le fait qu'elle comprend en outre au moins un polymère épaississant choisi dans le groupe constitué par :
   (ii) les homopolymères d'acide acrylique réticulés ;
   (iii) les copolymères réticulés d'acide (méth)acrylique et d'acrylate d'alkyle(C₁-C₆)
   (iv) les homopolymères et copolymères non-ioniques contenant des monomères à insaturation éthylénique de type ester et/ou amide ;
   (v) les homopolymères réticulés d'acide 2-acrylamido-2-méthyl-propane sulfonique et leurs copolymères réticulés d'acrylamide, partiellement ou totalement neutralisés ;
   (vi) les homopolymères d'acrylate d'ammonium ou les copolymères d'acrylate d'ammonium et d'acrylamide ;

La composition tinctoriale conforme à l'invention ainsi définie, conduit après mélange avec une composition oxydante, à des colorations dans des nuances variées, chromatiques, puissantes, esthétiques, présentant une faible sélectivité et d'excellentes propriétés de résistances à la fois vis-à-vis des agents atmosphériques tels que la lumière et les intempéries et vis-à-vis de la transpiration et des différents traitements que peuvent subir les cheveux.

Un autre objet de l'invention porte sur une composition prête à l'emploi pour la teinture d'oxydation des fibres kératiniques qui comprend, dans un milieu approprié pour la teinture, au moins une 3,5-diamino-pyridine de formule (1), au moins un précurseur de colorant d'oxydation, au moins un polymère épaississant ci-dessus défini et au moins un agent oxydant, à l'exclusion de la 2,6-diméthoxy-3,5-diamino-pyridine associée au terpolymère acide méthacrylique/acrylate d'éthyle/méthacrylate de stéaryle oxyéthyléné avec un système oxydant enzymatique à l'uricase.
Par composition prête à l'emploi, on entend au sens de la présente invention, toute composition destinée à être appliquée immédiatement sur les fibres kératiniques.

L'invention vise également un procédé de teinture d'oxydation des fibres kératiniques, et en particulier des fibres kératiniques humaines telles que les cheveux, consistant à appliquer sur les fibres une composition colorante contenant dans un milieu approprié pour la teinture, au moins une 3,5-diamino-pyridine de formule (I) et au moins un précurseur de colorant d'oxydation, la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère épaississant défini selon l'invention étant présent dans la composition colorante et/ou oxydante.

L'invention a également pour objet des dispositifs de teinture à plusieurs compartiments ou " kits "pour la teinture d'oxydation des fibres kératiniques, en particulier des fibres kératiniques humaines telles que les cheveux,
De tels dispositifs comportent un premier compartiment contenant au moins une 3,5-diamino-pyridine de formule (I) et au moins un précurseur de colorant d'oxydation et un deuxième compartiment contenant un agent oxydant, au moins un polymère épaississant défini selon l'invention, étant présent dans le premier compartiment et/ou dans le second compartiment.

Un autre dispositif de teinture à plusieurs compartiments comporte au moins un compartiment contenant au moins une 3,5-diamino-pyridine de formule (I) et au moins un précurseur de colorant d'oxydation, au moins un compartiment contenant au moins un polymère épaississant défini selon l'invention, et au moins un autre compartiment contenant au moins un agent oxydant.

Mais d'autres caractéristiques, aspects, objets et avantages de l'invention apparaîtront encore plus clairement à la lecture de la description et des exemples qui suivent.

Parmi les dérivés de 3,5-diaminopyridine de formule (I) conformes à l'invention, on peut citer la 2,6-diméthoxy-3,5-diamino-pyridine, la 2,6-diéthoxy-3,5-diamino-pyridine, la 2,6-di- (β-hydroxyéthyloxy)-3,5-diamino-pyridine et leurs sels d'addition avec un acide.

Selon l'invention, la composition tinctoriale renferme de préférence la 2,6-diméthoxy-3,5-diamino-pyridine, et/ou au moins l'un de ses sels d'addition avec un acide.

Le ou les dérivés de 3,5-diamino-pyridine de formule (I) utilisables dans la composition tinctoriale conforme à l'invention représentent de préférence de 0,0001 à 10 % en poids environ du poids total de la composition tinctoriale, et encore plus préférentiellement de 0,005 à 5 % en poids environ de ce poids.

La composition tinctoriale conforme à l'invention renferme au moins un précurseur de colorant d'oxydation ou base d'oxydation.
La nature de ces bases d'oxydation n'est pas critique.
Elles peuvent notamment être choisies parmi les paraphénylènediamines, les bases doubles, les para-aminophénols, les ortho-aminophénols et les bases d'oxydation hétérocycliques.

Parmi les paraphénylènediamines utilisables à titre de base d'oxydation dans les composition tinctoriales conformes à l'invention, on peut notamment citer les composés de formule (II) suivante et leurs sels d'addition avec un acide : 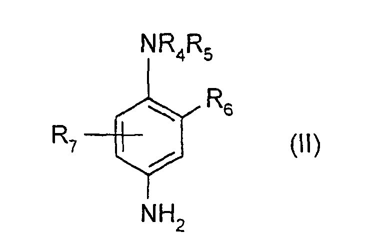 dans laquelle :
- R₄ représente un atome d'hydrogène, un radical alkyle en C₁-C₄, monohydroxyalkyle en C₁-C₄, polyhydroxyalkyle en C₂-C₄, alcoxy(C₁₋C₄)alkyle(C₁-C₄), alkyle en C₁-C₄ substitué par un groupement azoté, phényle ou 4'-aminophényle ;
- R₅ représente un atome d'hydrogène, un radical alkyle en C₁-C₄, monohydroxyalkyle en C₁-C₄, polyhydroxyalkyle en C₂-C₄, alcoxy(C₁₋C₄)alkyle(C₁-C₄) ou alkyle en C₁-C₄ substitué par un groupement azoté ;
- R₅ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₇ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 2-méthyl-1-N-(β-hydroxyéthyl) paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide : 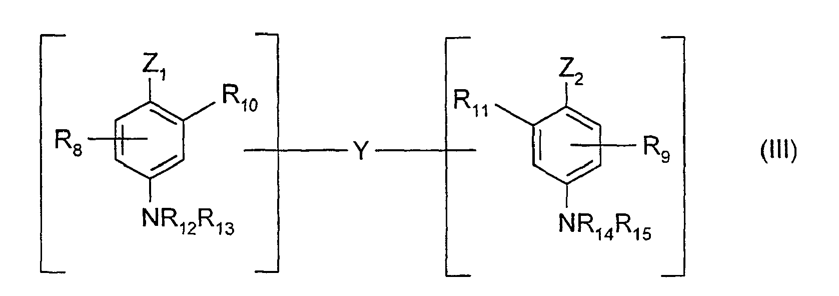 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un
ou plusieurs radicaux hydroxyle ou alcoxy en C₁-C₆ ;
- R₈ et R₉ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₀, R₁₁, R₁₂, R₁₃, R₁₄ et R₁₅, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylèpediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (IV) suivante, et leurs sels d'addition avec un acide : 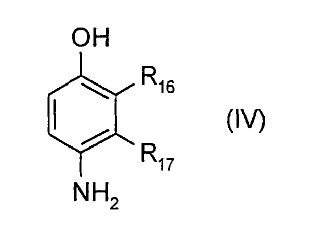 dans laquelle :
- R₁₆ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄
ou hydroxyalkyl(C₁-C₄)aminoalkyle en C₁-C₄,
- R₁₇ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)-amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique et leurs sels d'addition avec un acide.

Parmi les dérivés pyrazoliques, on peut plus particulièrement citer les composés décrits dans les brevets DE-3 843 892, DE-4 133 957 et demandes de brevet WO-94/08969, WO-94/08970, FR-A-2 733 749 et DE-195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon une forme de réalisation préférée de l'invention la ou les bases d'oxydation sont choisies parmi les paraphénylènediamines. La ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids.

### Polymères épaississants selon l'invention.

(ii) Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.

(iii) Parmi les copolymères réticulés d'acide (méth)acrylique et d'acrylate d'alkyle en C₁-C₆, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active.

(iv) Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle / diméthacrylate d'éthylèneglycol); ACRYLOID B66 par la société RHOM & HAAS (copolymère méthacrylate de butyle / méthacrylate de méthyle); BPA 500 par la société KOBO (polyméthacrylate de méthyle).

(v) Parmi les homopolymères réticulés d'acide 2-acrylamido-2-méthyl-propane sulfonique, on peut citer ceux décrits dans la demande EP-A-0815828 (faisant partie intégrante du contenu de la description). Parmi les copolymères réticulés d'acide 2-acrylamido-2-méthyl-propane sulfonique et d'acrylamide partiellement ou totalement neutralisés (par une base telle que la soude, de la potasse ou une amine) on peut citer en particulier le produit décrit dans l'exemple 1 du document EP-A-503 853 (faisant partie intégrante du contenu de la description).

(vi) Parmi les homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).

Le ou les polymères épaississants ainsi définis et utilisables dans les compositions tinctoriales de la présente invention sont présents de préférence à raison de 0,01 à 10% environ en poids, en particulier à raison de 0,1 à 5% environ en poids par rapport au poids total de la composition .
Selon la présente invention, les classes de polymères épaississants plus particulièrement préférées sont choisies parmi les classes répertoriées ci-dessus (ii), (iii), (v),
La composition de teinture selon l'invention peut également contenir un ou plusieurs coupleurs différant des 3,5-diamino-pyridines de formule (I) selon l'invention et choisis parmi les métaphénylènediamines, les méta-aminophénols, les métadiphénols, les naphtols et les coupleurs hétérocycliques tels que les pyrazolo-[1,5-b]-1,2,4-triazoles, les pyrazolo-[3,2-c]-1,2,4-triazoles, les pyrazol-5-ones, les pyridines différentes des 3,5-diamino-pyridines de formule (I) selon l'invention, les indoles, les indolines, les indazoles, les benzimidazoles, les benzothiazoles, les benzoxazoles, les 1,3-benzodioxoles et les quinolines, et leurs sels d'addition avec un acide.
Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole, le 3-(4-hydroxy-1-méthyl-1-H-indol-5-ylméthyl)-1-méthyl pyridinium, et leurs sels d'addition avec un acide.

Lorsqu'ils sont présents, le ou les coupleurs additionnels représentent de préférence de 0,0001 à 10 % en poids environ du poids total de la composition tinctoriale conforme à l'invention et encore plus préférentiellement de 0,005 à 5 % en poids environ de ce poids.

Les sels d'addition avec un acide des 3,5-diamino-pyridines de formule (I), des précurseurs de colorant d'oxydation et des coupleurs additionnels éventuellement présents et utilisables dans les compositions tinctoriales selon l'invention sont notamment choisis parmi les chlorhydrates, les bromhydrates, les sulfates, les tartrates, les lactates et les acétates.

La composition de teinture selon l'invention peut également contenir des colorants directs utilisés notamment pour modifier les nuances en les enrichissant de reflets; ils peuvent alors être choisis parmi les colorants nitrés azoïques ou anthraquinoniques, neutres, cationiques ou anioniques dans la proportion pondérale d'environ 0,001 à 20% et de préférence de 0,01 à 10% du poids total de la composition.

Le milieu de la composition approprié pour la teinture est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, des conservateurs, des céramides, des pseudocéramides, des huiles végétales, minérales ou de synthèse, les vitamines ou provitamines comme le panthénol, des opacifiants, ou des polymères autres que ceux de l'invention.
De préférence, la composition selon l'invention contient un polymère cationique.

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras peuvent représenter de 0,001 à 20% en poids environ du poids total de la composition.

De préférence, la composition colorante et/ou la composition oxydante de la composition prête à l'emploi selon l'invention contient au moins un tensioactif nonionique, anionique, cationique ou amphotère dans la proportion d'environ 0,1 à 20% en poids.
Encore plus préférentiellement ladite composition contient au moins un tensioactif nonionique.

Bien entendu, l'homme de l'art veillera à choisir le ou les éventuels composés complémentaires mentionnés ci-avant, de manière telle que les propriétés avantageuses attachées intrinsèquement à la composition de teinture d'oxydation selon l'invention ne soient pas, ou substantiellement pas, altérées par la ou les adjonctions envisagées.

Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons, le cas échéant en présence de leur donneur ou cofacteur respectif.

Le pH de la composition colorante ou de la composition prête à l'emploi et appliquée sur les fibres kératiniques [composition résultant du mélange de la composition colorante selon l'invention et de la composition oxydante], est généralement compris entre les valeurs 3 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium
ou de potassium et les composés de formule (VIII) suivante : 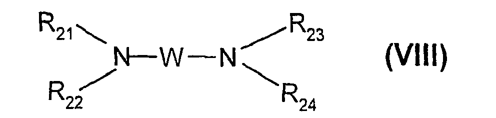 dans laquelle, W est un reste propylène éventuellement substitué par un groupement hydroxyle ou un radical alkyle en C₁-C₄ ; R₂₁, R₂₂, R₂₃ et R₂₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux
ou organiques comme l'acide chlorhydrique, l'acide orthophosphorique, des acides carboxyliques comme l'acide tartrique, l'acide citrique, l'acide lactique, ou des acides sulfoniques.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

Le procédé de teinture selon l'invention consiste, de préférence, à appliquer la composition prête à l'emploi, réalisée extemporanément au moment de l'emploi à partir de la composition colorante selon l'invention et de la composition oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Les exemples qui suivent sont destinés à illustrer l'invention.

### EXEMPLES 1 à 3

On a préparé les 3 compositions tinctoriales suivantes :
(exprimées en grammes - MA* désigne Matière Active)

| | **Exemple 1 comparatif** | **Exemple 2** | **Exemple 3 comparatif** |
|---|---|---|---|
| Dichlorhydrate de 2,6-diméthoxy-3,5-diamino-pyridine (coupleur de formule (I) | 0,363 | 0,484 | 0,605 |
| Paraphénylènediamine (base d'oxydation) | 0,324 | | |
| Sulfate de N,N-bis(β-hydroxyéthyl)-para-phénylènediamine (base d'oxydation) | | 0,936 | |
| Dichlorhydrate de 2-(β-hydroxyéthyl)-paraphénylènediamine (base d'oxydation) | | | 1,125 |
| Dichlorhydrate de 2,4-diamino-1-(β-hydroxy-éthyloxy)-benzène (coupleur additionnel) | 0,361 | 0,241 | 0,602 |
| Aculyn 44 vendu par la société ROHM & HAAS (polymère amphiphile non-ionique à chaîne grasse) | 4 | | |
| Carbopol 980 vendu par la société GOODRICH (acide polyacrylique réticulé) | | 1 | |
| Jaguar HP105 vendu par la société RHODIA CHIMIE (hydroxypropylguar) | | | 2 |
| Support de teinture commun | (*) | (*) | (*) |

| | | | |
|---|---|---|---|
| (*) Support de teinture commun | | | |

- Lauryl éther sulfate de sodium oxyéthyléné à 2,2 moles d'oxyde d'éthylène en solution aqueuse à 70%, vendu sous la dénomination TEXAPON NSW par la société GOGNIS 6,44 MA*
- Monoéthanolamide d'acide alkyl(C13/C15 70/30 50% linéaire) éther carboxylique oxyéthyléné à 2 moles d'oxyde d'éthylène 5,0
- Sel pentasodique de l'acide diéthylène triamine pentacétique en solution aqueuse à 40%, vendu sous la dénomination
   DISSOLUINE D-40 ® par la société AKZO 0,80 MA*
- Thiolactate d'ammonium en solution aqueuse à 58% 0,46 MA*
- Ammoniaque à 20,5% de NH₃ 12,0
- Eau déminéralisée qsp 100

Au moment de l'emploi, on a mélangé poids pour poids chacune des compositions tinctoriales décrites ci-dessus avec une solution de peroxyde d'hydrogène à 20 volumes (6% en poids) de pH 3.

Les mélanges ainsi réalisés ont été appliqués pendant 30 minutes sur des mèches de cheveux gris naturels permanentés à 90 % de blancs. Les mèches ont ensuite été rincées, lavées avec un shampooing standard, rincées à nouveau puis séchées. Elles ont été teintes dans les nuances réunies dans le tableau ci-dessous :

| EXEMPLES | Nuance |
|---|---|
| 1 | Brun naturel cendré |
| 2 | Châtain naturel cendré |
| 3 | Brun naturel cendré |

## Revendications

1. Composition pour la teinture d'oxydation des fibres kératiniques, en particulier des fibres kératiniques humaines telles que les cheveux, comprenant, dans un milieu approprié pour la teinture,
(a) **à titre de coupleur**, au moins un dérivé de 3,5-diamino-pyridine de formule (I) suivante : 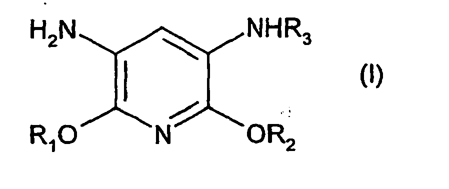 dans laquelle :
- R₁ et R₂, identiques ou différents, représentent un radical alkyle en C₁-C₄, monohydroxyalkyle en C₁-C₄, ou polyhydroxyalkyle en C₂-C₄,
- R₃ représente un atome d'hydrogène, un radical alkyle en C₁-C₄, monohydroxyalkyle en C₁-C₄ ou polyhydroxyalkyle en C₂-C₄
ou l'un de ses sels d'addition avec un acide ;
et,
(b) au moins un précurseur de colorant d'oxydation,
**caractérisée par le fait qu'**elle comprend en outre au moins un polymère épaississant choisi dans le groupe constitué par :
(ii) les homopolymères d'acide acrylique réticulés ;
(iii) les copolymères réticulés d'acide (méth)acrylique et d'acrylate d'alkyle(C₁-C₆)
(iv) les homopolymères et copolymères non-ioniques contenant des monomères à insaturation éthylénique de type ester et/ou amide ;
(v) les homopolymères réticulés d'acide 2-acrylamido-2-méthyl-propane sulfonique et leurs copolymères réticulés d'acrylamide, partiellement ou totalement neutralisés ;
(vi) les homopolymères d'acrylate d'ammonium ou les copolymères d'acrylate d'ammonium et d'acrylamide ;

2. Composition selon la revendication 1, **caractérisée par le fait que** les 3,5-diamino-pyridines de formule (I) sont choisies parmi la 2,6-diméthoxy-3,5-diamino-pyridine, la 2,6-diéthoxy-3,5-diamino-pyridine, la 2,6-di-(β-hydroxyéthytoxy)-3,5-diamino-pyridine et leurs sels d'addition avec un acide.

3. Composition selon la revendication 2, **caractérisée par le fait que** la 3,5-diamino-pyridine est la 2,6-diméthoxy-3,5-diamino-pyridine ou l'un de ses sels d'addition avec un acide.

4. Composition selon l'une quelconque des revendications précédentes, **caractérisée par le fait que** les 3,5-diamino-pyridines de formule (I) et leurs sels d'addition acide représentent de 0,0001 à 10% en poids du poids total de la composition.

5. Composition selon la revendication 4, **caractérisée par le fait que** les 3,5-diamino-pyridines de formule (I) et leurs sels d'addition acide représentent de 0,005 à 5% en poids du poids total de la composition.

6. Composition selon la revendication 1, **caractérisée par le fait que** le précurseur de colorant d'oxydation est choisi parmi les paraphénylènediamines, les bases doubles, les para-aminophénols, les ortho-aminophénols et les bases d'oxydation hétérocycliques et leurs sels d'addition avec un acide.

7. Composition selon l'une quelconque des revendications précédentes, **caractérisée par le fait que** le ou les précurseurs de colorant d'oxydation représentent de 0,0005 à 12% en poids du poids total de la composition.

8. Composition selon l'une quelconque des revendications 1 à 7, **caractérisée par le fait que** le polymère épaississant est choisi parmi les homopolymères d'acide acrylique réticulés.

9. Composition selon l'une quelconque des revendications 1 à 7, **caractérisée par le fait que** le polymère épaississant est choisi parmi les copolymères réticulés d'acide (méth)acrylique et d'acrylate d'alkyle(C₁-C₆).

10. Composition selon l'une quelconque des revendications 1 à 7, **caractérisée par le fait que** le polymère épaississant est choisi parmi les homopolymères réticulés d'acide 2-acrylamido-2-méthyl-propane sulfonique et leurs copolymères réticulés d'acrylamide, partiellement ou totalement neutralisés.

11. Composition selon l'une quelconque des revendications précédentes, **caractérisée par le fait que** le ou les polymères épaississants représentent de 0,01 à 10 % en poids du poids total de la composition.

12. Composition selon la revendication 11, **caractérisée par le fait que** la ou les polymères épaississants représentent de 0,1 à 5% en poids du poids total de la composition.

13. Composition selon l'une quelconque des revendications précédentes, **caractérisée par le fait qu'**elle contient au moins un coupleur additionnel.

14. Composition selon la revendication 13, **caractérisée par le fait que** les coupleurs sont choisis parmi les métaphénylènediamines, les métaaminophénols, les métadiphénols, les naphtols, les coupleurs hétérocycliques différents des 3,5-diamino-pyridines de formule (I), et les sels d'addition de ces composés avec un acide.

15. Composition selon l'une quelconque des revendications précédentes, **caractérisée par le fait que** les coupleurs sont présents dans des concentrations allant de 0,0001 à 10% en poids par rapport au poids total de la composition.

16. Composition selon l'une quelconque des revendications précédentes, **caractérisée par le fait que** les sels d'addition avec un acide des 3,5-diamino-pyridines de formule (I), des précurseurs de colorant d'oxydation et des coupleurs addtionnels sont choisis parmi les chlorhydrates, les bromhydrates, les sulfates, les tartrates, les lactates et les acétates.

17. Composition selon l'une quelconque des revendications précédentes, **caractérisée par le fait qu'**elle contient en outre au moins un colorant direct dans la proportion de 0,001 à 20% en poids par rapport au poids total de la composition.

18. Composition selon l'une quelconque des revendications précédentes, **caractérisée par le fait qu'**elle contient en outre au moins un agent réducteur ou antioxydant, dans des quantités allant de 0,05 à 1,5% en poids par rapport au poids total de la composition.

19. Composition prête à l'emploi pour la teinture d'oxydation des fibres kératiniques en particulier des fibres kératiniques humaines telles que les cheveux **caractérisée par le fait qu'**elle est obtenue par mélange d'une composition colorante telle que définie à l'une quelconque des revendications 1 à 18 et d'une composition oxydante contenant au moins un agent oxydant, à l'exclusion du coupleur 2,6-diméthoxy-3,5-diamino-pyridine associé au terpolymère acide méthacrylique/acrylate d'éthyle/méthacrylate de stéaryle oxyéthyléné dans un système oxydant enzymatique à l'uricase.

20. Composition selon la revendication 19, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

21. Composition selon la revendication 20, **caractérisée par le fait que** l'agent oxydant est le peroxyde d'hydrogène.

22. Composition selon la revendication 21, **caractérisée par le fait que** l'agent oxydant est une solution d'eau oxygénée dont le titre varie de 1 à 40 volumes.

23. Composition selon l'une quelconque des revendications précédentes, **caractérisée par le fait qu'**elle possède un pH allant de 3 à 12.

24. Composition selon la revendication 19, **caractérisée par le fait que** la composition colorante et/ou la composition oxydante contient au moins un tensioactif choisi parmi les tensioactifs anioniques, cationiques, non ioniques ou amphotères dans la proportion de 0,1 à 20% en poids par rapport au poids total de la composition.

25. Procédé de teinture d'oxydation des fibres kératiniques, et en particulier des fibres kératiniques humaines telles que les cheveux, consistant à appliquer sur les fibres une composition colorante contenant, dans un milieu approprié pour la teinture, au moins une 3,5-diamino-pyridine de formule (I) telle que définie à l'une quelconque des revendications 1 à 6, la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère épaississant tel que défini à l'une quelconque des revendications 1 et 8 à 12 étant présent dans la composition colorante et/ou oxydante.

26. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins deux compartiments, dont l'un d'entre eux contient au moins une 3,5-diamino-pyridine de formule (I) telle que définie à l'une quelconque des revendications 1 à 6, et un deuxième compartiment contenant au moins un agent oxydant, au moins un polymère épaississant tel que défini à l'une quelconque des revendications 1 et 8 à 12 étant présent dans le premier compartiment et/ou dans le second compartiment.

27. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins un compartiment contenant au moins une 3,5-diamino-pyridine de formule (I) telle que définie à l'une quelconque des revendications 1 à 6, au moins un compartiment contenant au moins un polymère épaississant tel que défini à l'une quelconque des revendications 1 et 8 à 12, et au moins un autre compartiment contenant au moins un agent oxydant.

## Claims

1. Composition for the oxidation dyeing of keratin fibres, in particular of human keratin fibres such as the hair, comprising, in a medium that is suitable for dyeing:
(a) **as coupler**, at least one 3,5-diaminopyridine derivative of formula (I) below: 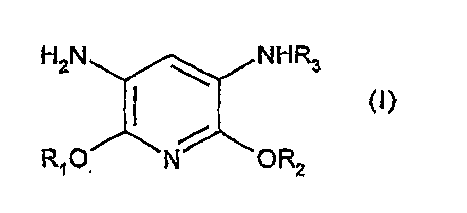 in which
- R₁ and R₂, which may be identical or different, represent a C₁-C₄ alkyl, C₁-C₄ monohydroxyalkyl or C₂-C₄ polyhydroxyalkyl radical,
- R₃ represents a hydrogen atom or a C₁-C₄ alkyl, C₁-C₄ monohydroxyalkyl or C₂-C₄ polyhydroxyalkyl radical,
or one of the addition salts thereof with an acid;
and
(b) at least one oxidation dye precursor,
**characterized in that** it also comprises at least one thickening polymer chosen from the group consisting of:
(ii) crosslinked acrylic acid homopolymers;
(iii) crosslinked copolymers of (meth)acrylic acid and of a (C₁-C₆) alkyl acrylate;
(iv) nonionic homopolymers and copolymers containing ethylenically unsaturated monomers of ester and/or amide type;
(v) crosslinked homopolymers of 2-acrylamido-2-methylpropanesulphonic acid and the partially or totally neutralized crosslinked acrylamide copolymers thereof;
(vi) ammonium acrylate homopolymers or copolymers of ammonium acrylate and of acrylamide.

2. Composition according to Claim 1, **characterized in that** the 3,5-diaminopyridines of formula (I) are chosen from 2,6-dimethoxy-3,5-diaminopyridine, 2,6-diethoxy-3,5-diaminopyridine and 2,6-di (β-hydroxyethyloxy)-3,5-diaminopyridine, and the addition salts thereof with an acid.

3. Composition according to Claim 2, **characterized in that** the 3,5-diaminopyridine is 2,6-dimethoxy-3,5-diaminopyridine, or an addition salt thereof with an acid.

4. Composition according to any one of the preceding claims, **characterized in that** the 3,5-diaminopyridines of formula (I) and the addition salts thereof with an acid represent from 0.0001% to 10% by weight relative to the total weight of the composition.

5. Composition according to Claim 4, **characterized in that** the 3,5-diamirlopyridines of formula (I) and the addition salts thereof with an acid represent from 0.005% to 5% by weight relative to the total weight of the composition.

6. Composition according to Claim 1, **characterized in that** the oxidation dye precursor is chosen from para-phenylenediamines, double bases, para-aminophenols, ortho-aminophenols and heterocyclic oxidation bases, and the addition salts thereof with an acid.

7. Composition according to any one of the preceding claims, **characterized in that** the oxidation dye precursor(s) represent(s) from 0.0005% to 12% by weight relative to the total weight of the composition.

8. Composition according to any one of Claims 1 to 7, **characterized in that** the thickening polymer is chosen from crosslinked acrylic acid homopolymers.

9. Composition according to any one of Claims 1 to 7, **characterized in that** the thickening polymer is chosen from crosslinked copolymers of (meth)acrylic acid and of a (C₁-C₆) alkyl acrylate.

10. Composition according to any one of Claims 1 to 7, **characterized in that** the thickening polymer is chosen from crosslinked 2-acrylamido-2-methylpropanesulphonic acid homopolymers and partially or totally neutralized crosslinked acrylamide copolymers thereof.

11. Composition according to any one of the preceding claims, **characterized in that** the thickening polymer(s) represent(s) from 0.01% to 10% by weight relative to the total weight of the composition.

12. Composition according to Claim 11, **characterized in that** the thickening polymer(s) represent(s) from 0.1% to 5% by weight relative to the total weight of the composition.

13. The composition according to any one of the preceding claims, **characterized in that** it contains at least one additional coupler.

14. Composition according to Claim 13, **characterized in that** the couplers are chosen from metaphenylenediamines, meta-aminophenols, metadiphenols, naphthols and heterocyclic couplers other than the 3,5-diaminopyridines of formula (I), and the addition salts of these compounds with an acid.

15. Composition according to any one of the preceding claims, **characterized in that** the couplers are present in concentrations ranging from 0.0001% to 10% by weight relative to the total weight of the composition.

16. Composition according to any one of the preceding claims, **characterized in that** the addition salts with an acid of the 3,5-diaminopyridines of formula (I), of the oxidation dye precursors and of the additional couplers are chosen from the hydrochlorides, hydrobromides, sulphates, tartrates, lactates and acetates.

17. Composition according to any one of the preceding claims, **characterized in that** it also contains at least one direct dye in a proportion of from 0.001% to 20% by weight relative to the total weight of the composition.

18. Composition according to any one of the preceding claims, **characterized in that** it also contains at least one reducing agent or antioxidant, in amounts ranging from 0.05% to 1.5% by weight relative to the total weight of the composition.

19. Ready-to-use composition for the oxidation dyeing of keratin fibres, in particular of human keratin fibres such as the hair, **characterized in that** it is obtained by mixing together a dye composition as defined in any one of Claims 1 to 18 and an oxidizing composition containing at least one oxidizing agent, with the exclusion of the coupler 2,6-dimethoxy-3,5-diaminopyridine combined with the methacrylic acid/ethyl acrylate/stearyl methacrylate oxyethylenated terpolymer in a uricase-containing enzymatic oxidizing system.

20. Composition according to Claim 19, **characterized in that** the oxidizing agent is chosen from hydrogen peroxide, urea peroxide, alkali metal bromates or ferricyanides, persalts, redox enzymes such as laccases, peroxidases and 2-electron oxidoreductases, where appropriate in the presence of the respective donor or cofactor thereof.

21. Composition according to Claim 20, **characterized in that** the oxidizing agent is hydrogen peroxide.

22. Composition according to Claim 21, **characterized in that** the oxidizing agent is an aqueous hydrogen peroxide solution with a titre ranging from 1 to 40 volumes.

23. Composition according to any one of the preceding claims, **characterized in that** it has a pH ranging from 3 to 12.

24. Composition according to Claim 19, **characterized in that** the dye composition and/or the oxidizing composition contains at least one surfactant chosen from anionic, cationic, nonionic and amphoteric surfactants in a proportion of from 0.1% to 20% by weight relative to the total weight of the composition.

25. Process for the oxidation dyeing of keratin fibres, and in particular of human keratin fibres such as the hair, which consists in applying to the fibres a dye composition containing, in a medium that is suitable for dyeing, at least one 3,5-diaminopyridine of formula (I) as defined in any one of Claims 1 to 6, the colour being developed at alkaline, neutral or acidic pH with the aid of an oxidizing composition that is mixed with the dye composition just at the time of use, or that is applied sequentially without intermediate rinsing, at least one thickening polymer as defined in any one of Claims 1 and 8 to 12 being present in the dye composition and/or oxidizing composition.

26. Multi-compartment device or kit for the oxidation dyeing of keratin fibres, and in particular of human keratin fibres such as the hair, **characterized in that** it comprises at least two compartments, one of which contains at least one 3,5-diaminopyridine of formula (I) as defined in any one of Claims 1 to 6, and a second compartment containing at least one oxidizing agent, at least one thickening polymer as defined in any one of Claims 1 and 8 to 12 being present in the first compartment and/or in the second compartment.

27. Multi-compartment device or kit for the oxidation dyeing of keratin fibres, and in particular of human keratin fibres such as the hair, **characterized in that** it comprises at least one compartment containing at least one 3,5-diaminopyridine of formula (I) as defined in any one of Claims 1 to 6, at least one compartment containing at least one thickening polymer as defined in any one of Claims 1 and 8 to 12, and at least one other compartment containing at least one oxidizing agent.

## Patentansprüche

1. Zusammensetzung zum oxidativen Färben von Keratinfasern und insbesondere menschlichen Keratinfasern, wie Haaren, die in einem zum Färben geeigneten Medium enthält:
(a) **als Kuppler** mindestens ein 3,5-Diaminopyridinderivat der folgenden Formel (I): 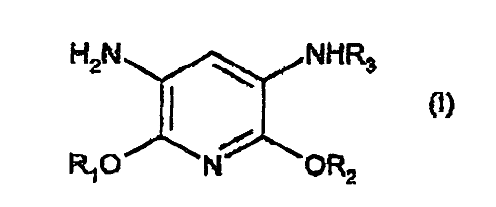 worin bedeuten:
- die Gruppen R₁ und R₂, die gleich oder verschieden sind, eine C₁₋₄-Alkylgruppe, eine C₁₋₄-Monohydroxyalkylgruppe oder eine C₂₋₄-Polyhydroxyalkylgruppe;
- R₃ ein Wasserstoffatom, eine C₁₋₄-Alkylgruppe, eine C₁₋₄₋Monohydroxyalkylgruppe oder eine C₂₋₄-Polyhydroxyalkylgruppe,
oder mindestens eines seiner Additionssalze mit einer Säure; und
b) mindestens ein Farbstoffvorprodukt eines Oxidationsfarbstoffes,
**dadurch gekennzeichnet, dass** sie ferner mindestens ein verdickendes Polymer enthält, das unter den folgenden Verbindungen ausgewählt ist:
**(ii)** vernetzten Homopolymeren von Acrylsäure;
**(iii)** vernetzten Copolymeren von (Meth)acrylsäure und Alkyl(C₁₋₆)acrylat;
**(iv)** nichtionischen Homopolymeren und Copolymeren, die Monomere vom Estertyp und/oder Amidtyp mit ethylenisch ungesättigter Bindung enthalten;
**(v)** vernetzten Homopolymeren von 2-Acrylamido-2-methyl-propansulfonsäure und ihren vernetzten Copolymeren mit Acrylamid, die ganz oder teilweise neutralisiert sind;
**(vi)** Homopolymeren von Ammoniumacrylat oder Copolymeren von Ammoniumacrylat und Acrylamid.

2. Zusammensetzung nach Anspruch 1, **dadurch gekennzeichnet, dass** die 3,5-Diaminopyridine der Formel (I) unter 2,6-Dimethoxy-3,5-diaminopyridin, 2,6-Diethoxy-3,5-diaminopyridin, 2,6-Di-(β-hydroxyethyloxy)-3,5-diaminopyridin und deren Additionssalzen mit einer Säure ausgewählt sind.

3. Zusammensetzung nach Anspruch 2, **dadurch gekennzeichnet, dass** das 3,5-Diaminopyridin das 2,6-Dimethoxy-3,5-diaminopyridin oder eines seiner Additionssalze mit einer Säure ist.

4. Zusammensetzung nach einem der vorhergehenden Ansprüche, **dadurch gekennzeichnet, dass** die 3,5-Diaminopyridine der Formel (I) und deren Additionssalze mit einer Säure 0,0001 bis 10 Gew.-% des Gesamtgewichts der Zusammensetzung ausmachen.

5. Zusammensetzung nach Anspruch 4, **dadurch gekennzeichnet, dass** die 3,5-Diaminopyridine der Formel (I) und deren Additionssalze mit einer Säure 0,005 bis 5 Gew.-% des Gesamtgewichts der Zusammensetzung ausmachen.

6. Zusammensetzung nach Anspruch 1, **dadurch gekennzeichnet, dass** das Farbstoffvorprodukt eines Oxidationsfarbstoffes unter den *p*-Phenylendiaminen, Doppelbasen, *p*-Aminophenolen, *o*-Aminophenolen und den heterocyclischen Oxidationsbasen und deren Additionssalzen mit einer Säure ausgewählt sind.

7. Zusammensetzung nach einem der vorhergehenden Ansprüche, **dadurch gekennzeichnet, dass** das Farbstoffvorprodukt eines Oxidationsfarbstoffes oder die Farbstoffvorprodukte von Oxidationsfarbstoffen 0,0005 bis 12 Gew.-% des Gesamtgewichts der Zusarrimensetzung ausmachen.

8. Zusammensetzung nach einem der Ansprüche 1 bis 7, **dadurch gekennzeichnet, dass** das verdickende Polymer unter den vernetzten Acrylsäure-Homopolymeren ausgewählt ist.

9. Zusammensetzung nach einem der Ansprüche 1 bis 7, **dadurch gekennzeichnet, dass** das verdickende Polymer unter den vernetzten Copolymeren von (Meth)acrylsäure und Alkyl(C₁₋₆)-acrylat ausgewählt ist.

10. Zusammensetzung nach einem der Ansprüche 1 bis 7, **dadurch gekennzeichnet, dass** das verdickende Polymer unter den vernetzten Homopolymeren von 2-Acrylamido-2-methylpropansulfonsäure und ihren vernetzten Copolymeren mit Acrylamid, die ganz oder teilweise neutralisiert sind, ausgewählt ist.

11. Zusammensetzung nach einem der vorhergehenden Ansprüche, **dadurch gekennzeichnet, dass** das oder die verdickende(n) Polymer(e) 0,01 bis 10 Gew.-% des Gesamtgewichts der Zusammensetzung ausmachen.

12. Zusammensetzung nach Anspruch 11, **dadurch gekennzeichnet, dass** das oder die verdickende(n) Polymer(e) 0,1 bis 5 Gew.-% des Gesamtgewichts der Zusammensetzung ausmachen.

13. Zusammensetzung nach einem der vorhergehenden Ansprüche, **dadurch gekennzeichnet, dass** sie mindestens einen zusätzlichen Kuppler enthält.

14. Zusammensetzung nach Anspruch 13, **dadurch gekennzeichnet, dass** die Kuppler unter den *m*-Phenylendiaminen, *m*-Aminophenolen, *m*-Dihydroxybenzolen, Naphtholen, heterocyclischen Kupplern, die von den 3,5-Diaminopyridinen der Formel (I) verschieden sind, und den Additionssalzen dieser Verbindungen mit einer Säure ausgewählt sind.

15. Zusammensetzung nach einem der vorhergehenden Ansprüche, **dadurch gekennzeichnet, dass** die Kuppler in Konzentrationen von 0,0001 bis 10 Gew.-%, bezogen auf das Gesamtgewicht der Zusammensetzung, enthalten sind.

16. Zusammensetzung nach einem der vorhergehenden Ansprüche, **dadurch gekennzeichnet, dass** die Additionssalze der 3,5-Diaminopyridine der Formel (1), der Farbstoffvorprodukte von Oxidationsfarbstoffen und der zusätzlichen Kuppler mit einer Säure unter den Hydrochloriden, Hydrobromiden, Sulfaten, Tartraten, Lactaten und Acetaten. ausgewählt sind.

17. Zusammensetzung nach einem der vorhergehenden Ansprüche, **dadurch gekennzeichnet, dass** sie ferner mindestens einen Direktfarbstoff in einem Mengenanteil von 0,001 bis 20 Gew.-%, bezogen auf das Gesamtgewicht der Zusammensetzung, enthält.

18. Zusammensetzung nach einem der vorhergehenden Ansprüche, **dadurch gekennzeichnet, dass** sie ferner mindestens ein Reduktionsmittel oder Antioxidationsmittel in Mengenanteilen von 0,05 bis 1,5 Gew.-%, bezogen auf das Gesamtgewicht der Zusammensetzung, enthält.

19. Gebrauchsfertige Zusammensetzung zum oxidativen Färben von Keratinfasern und insbesondere menschlichen Keratinfasern, wie Haaren, **dadurch gekennzeichnet, dass** sie durch Mischen einer Farbmittelzusammensetzung nach einem der Ansprüche 1 bis 18 und einer oxidierenden Zusammensetzung, die mindestens ein Oxidationsmittel enthält, hergestellt wird, mit der Maßgabe, dass der Kuppler 2,6-Dimethoxy-3,5-diaminopyridin in Kombination mit dem ethoxylierten Methacrylsäure/Ethylacrylat/Stearylmethacrylat-Terpolymer in einem enzymatischen. Oxidationssystem mit Uricase ausgenommen ist.

20. Zusammensetzung nach Anspruch 19, **dadurch gekennzeichnet, dass** das Oxidationsmittel unter Wasserstoffperoxid, Harnstoffperoxid, Alkalimetallbromaten oder -ferricyaniden, Salzen von Persäuren, Redoxenzymen, wie Laccasen, Peroxidasen oder O-xidoreduktasen (2 Elektronen) gegebenenfalls in Gegenwart des jeweiligen Donors oder Cofaktors ausgewählt ist.

21. Zusammensetzung nach Anspruch 20, **dadurch gekennzeichnet, dass** das Oxidationsmittel Wasserstoffperoxid ist.

22. Zusammensetzung nach Anspruch 21, **dadurch gekennzeichnet, dass** das Oxidationsmittel eine Wasserstoffperoxidlösung ist, deren Titer im Bereich von 1 bis 40 Volumina liegt.

23. Zusammensetzung nach einem der vorhergehenden Ansprüche, **dadurch gekennzeichnet, dass** sie einen pH-Wert von 3 bis 12 aufweist.

24. Zusammensetzung nach Anspruch 19, **dadurch gekennzeichnet, dass** die Farbmittelzusammensetzung und/oder die oxidierende Zusammensetzung mindestens einen grenzflächenaktiven Stoff enthält, der unter den anionischen, kationischen, nichtionischen oder amphoteren grenzflächenaktiven Stoffen in Mengenanteilen von 0,1 bis 20 Gew.-%, bezogen auf das Gesamtgewicht der Zusammensetzung, ausgewählt ist.

25. Verfahren zum oxidativen Färben von Keratinfasern und insbesondere menschlichen Keratinfasern, wie Haaren, das darin besteht, auf die Fasern ein Farbmittelzusammensetzung aufzutragen, die in einem zum Färben geeigneten Medium mindestens ein 3,5-Diaminopyridin der Formel (I) nach einem der Ansprüche 1 bis 6 enthält, wobei die Farbe bei einem alkalischen, neutralen oder sauren pH-Wert mit einer oxidierenden Zusammensetzung gebildet wird, die bei der Anwendung mit der Farbmittelzusammensetzung vermischt oder die getrennt davon anschließend ohne zwischenzeitliches Spülen aufgetragen wird, wobei mindestens ein verdickendes Polymer nach einem der Ansprüche 1 und 8 bis 12 in der Farbmittelzusammensetzung und/ oder in der oxidierenden Zusammensetzung enthalten ist.

26. Vorrichtung mit mehreren Abteilungen oder "Kit" zum oxidativen Färben von Keratinfasern und insbesondere menschlichen Keratinfasern, wie Haaren, **dadurch gekennzeichnet, dass** sie mindestens zwei Abteilungen aufweist, eine Abteilung, die mindestens ein 3,5-Diaminopyridin der Formel (I) nach einem der Ansprüche 1 bis 6 enthält, und eine zweite Abteilung, die mindestens ein Oxidationsmittel enthält, wobei mindestens ein verdickendes Polymer nach einem der Ansprüche 1, und 8 bis 12 in der ersten Abteilung und/oder in der zweiten Abteilung enthalten ist.

27. Vorrichtung mit mehreren Abteilungen oder "Kit" zum oxidativen Färben von Keratinfasern und insbesondere menschlichen Keratinfasern, wie Haaren, **dadurch gekennzeichnet, dass** sie mindestens eine Abteilung, die mindestens ein 3,5-Diaminopyridin der Formel (I) nach einem der Ansprüche 1 bis 6 enthält, mindestens eine Abteilung, die mindestens ein verdickendes Polymer nach einem der Ansprüche 1 und 8 bis 12 enthält, und mindestens eine weitere Abteilung aufweist, die mindestens ein Oxidationsmittel enthält.
